# FASCICULE DE BREVET EUROPEEN

(11) **EP 0 378 936 B1**
(45) Date de publication et mention de la délivrance du brevet: **14.07.1993**
(21) Numéro de dépôt: 89403474.3
(22) Date de dépôt: 14.12.1989
(51) Int. Cl.: A61K 7/48, A61K 31/60

(54) **Utilisation de dérivés salicylés pour le traitement du vieillissement de la peau**
Verwendung von Salicylsäure-Derivaten zur Behandlung des Hautalterns
Use of salicylic-acid derivatives in the treatment of aging of the skin

(30) Priorité: 16.12.1988 LU 87408
(43) Date de publication de la demande: 25.07.1990
(73) Titulaire: L'OREAL, 75008 Paris (FR)
(72) Inventeur: Leveque, Jean-Luc, F-93340 Le Raincy (FR); Saint Léger, Didier, F-75017 Paris (FR)
(74) Mandataire: Casalonga, Axel

(56) Documents cités:
- EP-A- 0 124 905
- FR-A- 2 581 542
- FR-A- 2 607 498

## Description

La présente invention a pour objet l'utilisation de compositions cosmétiques destinées à traiter le vieillissement de la peau en mettant en oeuvre des dérivés d'acide salicylique.

On constate au cours du processus de vieillissement l'apparition de différents signes au niveau de la peau, très caractéristiques de ce vieillissement, se traduisant notamment par une modification de la structure et de la fonction cutanée. Ces signes sont particulièrement marqués sur les zones découvertes, telles que le visage, les mains, auxquelles se rajoutent généralement des caractéristiques particulières dues à l'exposition de la peau à la lumière solaire (vieillissement actinique).

Il est difficile, en général, sur une zone découverte, de distinguer clairement entre les signes provenant du processus physiologique de vieillissement de la peau et entre les signes dus à l'exposition répétée de la peau à la lumière.

Les principaux signes cliniques de vieillissement cutané sont les suivants : apparition de rides profondes en augmentation avec l'âge. On constate en particulier une désorganisation du "grain" de la peau, c'est-à-dire que le micro-relief est moins régulier et présente un caractère anisotrope.

Le teint de la peau est généralement modifié, il apparaît plus pâle et plus jaune, ce qui semble être dû essentiellement à une désorganisation de la microcirculation (moins d'hémoglobine au niveau du derme papillaire). De nombreuses tâches colorées apparaissent en surface, ce qui est dû à une mélanogénèse altérée. Il existe sur certaines zones des irritations diffuses et parfois des telangiectasies.

Un autre signe clinique de vieillissement est l'aspect sec et rèche de la peau qui est dû essentiellement à une desquamation plus importante, ces squames en diffractant les rayons lumineux participent aussi à l'aspect un peu gris du teint.

On constate enfin une perte de fermeté et de tonicité de la peau qui, comme pour les rides, s'explique du moins en partie par une atrophie dermique et épidermique ainsi qu'un applatissement de la formation dermoépidermique.

On constate donc que les signes cliniques de vieillissement cutané résultent essentiellement d'un dysfonctionnement des principaux mécanismes biologiques intervenant au niveau de la peau.

La demanderesse a découvert de façon surprenante que l'utilisation de dérivés d'acide salicylique permettait de retarder ce vieillissement et d'agir sur les signes cliniques de celui-ci.

Elle a constaté plus particulièrement que grâce à ces dérivés salicyliques, il était possible d'atténuer pour le moins les rides, de modifier le teint de la peau qui apparaît plus rosé, d'effacer les taches pigmentées en surface, de supprimer les squames et de donner une consistance plus élastique à la peau. On a constaté en particulier qu'il était possible de conférer à la peau un toucher beaucoup plus doux.

Certains dérivés d'acide salicylique différents de ceux utilisables dans le cadre de l'invention, sont connus et décrits dans la demande de brevet européen 0 124 905, en tant qu'inhibiteurs de protéases, responsables de la dégradation des tissus naturels et plus particulièrement de la dégradation excessive de l'élastine.

Les dérivés d'acide salicylique utilisables conformément à l'invention, sont connus en eux-même et ont déjà été décrits dans la demande de brevet français 2581542 comme pouvant être utilisés dans des compositions topiques dans le domaine de la cosmétologie et la dermopharmacie et plus particulièrement comme agents thérapeutiques et comédolytiques, tels que pour le traitement de l'acné.

La demanderesse a découvert maintenant une application thérapeutique nouvelle agissant sur un mécanisme biologique totalement différent de celui de l'acné que l'on rencontre principalement chez les sujets jeunes.

L'invention a donc principalement pour objet l'utilisation pour la préparation d'un médicament destiné au traitement thérapeutique du vieillissement de la peau, de dérivés d'acide salicylique.

Un autre objet de l'invention est constitué par l'utilisation de compositions cosmétiques contenant ces dérivés d'acide salicylique pour le traitement du vieillissement de la peau.

D'autres objets de l'invention apparaîtront à la lecture de la description et des exemples qui suivent.

Les dérivés d'acide salicylique utilisables conformément à l'invention pour le traitement du vieillissement de la peau, répondent essentiellement à la formule générale suivante : 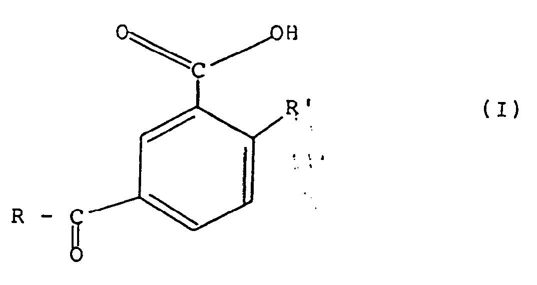
dans laquelle :
R représente une chaîne aliphatique saturée ayant de 3 à 11 atomes de carbone, linéaire, ramifiée ou cyclisé, une chaîne insaturée ayant de 3 à 17 atomes de carbone, portant une ou plusieurs doubles liaisons conjuguées ou non, les chaînes ci-dessus peuvent également être substituées par un ou plusieurs atomes d'halogène ainsi que par des groupements trifluorométhyle, par un ou plusieurs groupements hydroxyle sous forme libre ou estérifiée par un acide ayant de 1 à 6 atomes de carbone ou bien par une fonction carboxyle, libre ou estérifiée, par un alcool inférieur ayant de 1 à 6 atomes de carbone, ces différents groupements pouvant être simultanément présents sur lesdits substituants;
R' représente un groupement hydroxyle ou une fonction ester répondant à la formule : 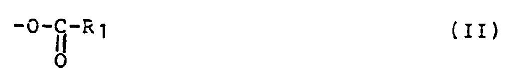
dans laquelle R₁ est un groupement aliphatique, saturé ou insaturé, ayant de 1 à 8 atomes de carbone.

Les composés plus particulièrement préférés sont ceux répondant à la formule (I) ci-dessus, dans lesquels R' désigne une fonction hydroxyle, R un groupement alkyle ayant de 3 à 11 atomes de carbone.

D'autres composés particulièrement intéressants sont ceux dans lesquels R représente une chaîne dérivée de l'acide linoléique, linolénique ou oléique.

Un autre groupe de composés particulièrement préférés est constitué par les composés dans lesquels R désigne une chaîne alkyle de 3 à 11 atomes de carbone et portant un groupement carboxylique libre, estérifié ou salifié et R' désigne une fonction hydroxyle.

Des composés particulièrement intéressants sont choisis parmi l'acide n-octanoyl-5 salicylique, l'acide n-dodécanoyl-5 salicylique, l'acide n-décanoyl-5 salicylique, ces composés pouvant éventuellement être utilisés sous forme micronisée.

Des essais effectués par la demanderesse ont permis en particulier de montrer que les composés utilisés conformément à l'invention ont un effet angiogénique particulièrement remarquable.

On constate que l'application de la composition à base d'acide n-octanoyl-5 salicylique provoque une large augmentation du volume et du nombre des capillaires.

Les compositions cosmétiques utilisées conformément à l'invention, dans le traitement du vieillissement cutané, contiennent dans un milieu physiologiquement acceptable, au moins un dérivé d'acide salicylique de formule (I) définie ci-dessus et se présentent généralement sous forme de gels, de crèmes, de lotions, de sticks, de mousses, de savons ou peuvent être préssurisées dans des dispositifs aérosols.

Elles peuvent contenir de l'eau et/ou des solvants compatibles avec la peau, tels que les alcools inférieurs en C₁-C₄, comme l'éthanol, isopropanol, des polyalcools tels que les propylèneglycol, la glycérine, ces solvants étant présents dans des proportions comprises entre 5 et 99% en poids.

Elles peuvent également se présenter sous forme de compositions grasses, contenant des huiles ou des corps gras, tels que la vaseline et les triglycérides.

Le composé de formule (I) est présent dans ces compositions de préférence dans des proportions comprises entre C,1 et 10% en poids.

Ces compositions cosmétiques peuvent également contenir des épaississants, des adoucissants, des surgraissants, des émollients, des mouillants, des agents de surface, des conservateurs, des agents anti-mousse, des filtres solaires, des huiles, des cires, des colorants et/ou des pigments ayant pour fonction de colorer la peau ou la composition elle-même, des anti-oxydants et tout autre ingrédients habituellement utilisé dans des compositions destinées à une application topique.

Des compositions cosmétiques particulièrement préférées sont des gels anhydres, des crèmes, des pommades grasses ou des laits comprenant en plus des composés définis ci-dessus, des alcools gras, des alcool gras oxyéthylénés ou polyglycérolés, des esters d'acides gras, des huiles naturelles ou synthétiques, des cires.

Dans le traitement du vieillissement cutané, ces compositions sont plus particulièrement appliquées sur les zones exposées à la lumière à raison de 1 à 10 mg/cm² de la peau et pendant une durée pouvant aller d'une semaine à 20 semaines ou plus.

Les compositions cosmétiques utilisées conformément à l'invention peuvent également contenir d'autres substances actives ayant un effet sur le vieillisement de la peau, telles que plus particulièrement des rétinoïdes, tels que par exemple l'acide rétinoïque tout-trans, l'acide 13-cis rétinoïque, le retinol, le rétinal et leurs dérivés. Il est également possible d'appliquer ces dérivés en deux temps de façon successive ou décalées dans le temps.

Les exemples suivants sont destinés à illustrer l'invention.

### EXEMPLE 1

On prépare la composition suivante :
- Propylèneglycol 45,00g
- Acide n-octanoyl-5- salicylique 3,00 g
- KLUCEL H (hydroxypropylcellulose) vendue par la Société HERCULES 1,50 g
- Ethanol anhydre qsp 100,00 g

Cette composition se présente sous forme de gel.

### EXEMPLE 2

On prépare la composition suivante :
- Méthyléther de propylèneglycol 37,00 g
- Hydroxypropylcellulose 1,50 g
- Acide n-octanoyl-5 salicylique 3,00 g
- Ethanol anhydre qsp 100,00 g

Cette composition se présente sous forme de gel.

### EXEMPLE 3

On prépare la composition suivante :
- Méthyléther de propylèneglycol 35,00 g
- Hydroxyéthylcellulose 1,50 g
- Acide n-octanoyl-5 salicylique 5,00 g
- Ethanol anhydre qsp 100,00 g

Cette composition se présente sous forme de gel.

### EXEMPLE 4

On prépare la composition suivante :
- Propylèneglycol 35,00 g
- Hydroxypropylcellulose 1,50 g
- Acide n-octanoyl-5 salicylique 5,00 g
- Ethanol anhydre qsp 100,00 g

Cette composition se présente sous forme de gel.

### EXEMPLE 5

On prépare la composition suivante :
- Vaseline 65,00 g
- Acide n-octanoyl-5 salicylique 5,00 g
- Perhydrosqualène qsp 100,00g

Cette composition est utilisable sous forme de pommade grasse.

### EXEMPLE 6

On prépare la composition suivante :
- Propylèneglycol 5,00 g
- Acide n-octanoyl-5 salicylique 4,00 g
- Acide acrylique réticulé par un agent polyfonctionnel, vendu sous la dénomination CARBOPOL 940 par la Société GOODRICH 0,80 g
- NaOH 0,125 g
- Eau qsp 100,00 g

L'acide n-octanoyl-5 salicylique est utilisé sous forme micronisée avec une granulométrie inférieure à 5 µ.

Cette composition se présente sous forme de gel.

### EXEMPLE 7

On prépare la composition suivante :
- Monostéarate de glycérol 0,80 g
- Alcool cétylique 2,00 g
- Alcool cétylstéarylique 5,00 g
- Stéarate de polyoxyéthylène, vendu par la Société ATLAS sous la dénomination commerciale de MYRJ 49 3,00 g
- Acide acrylique réticulé par un agent polyfonctionnel, vendu sous la dénomination CARBOPOL 941 par la Société GOODRICH 0,50 g
- Triéthanolamine 0,30 g
- MIGLYOL 810 vendu par la Société DYNAMIT NOBEL 12,00 g
- Conservateur qs
- Acide n-octanoyl-5 salicylique 6,00 g
- Eau qsp 100,00 g

Cette composition est utilisée comme crème hydratante.

### EXEMPLE 8

On prépare la composition suivante :
- Acide n-octanoyl-5 salicylique 4,00 g
- Polyéthylèneglycol 400 18,50 g
- Méthyléther de propylèneglycol 18,50 g
- Hydroxypropylcellulose 1,50 g
- Ethanol anhydre qsp 100,00 g

Cette composition se présente sous forme d'un gel.

### EXEMPLE 9

On prépare la composition suivante :
- Acide n-octanoyl-5 salicylique sous forme micronisée, ayant une granulométrie inférieure à 5 µ 3,00 g
- Propylèneglycol 6,00 g
- Acide acrylique réticulé par un agent polyfonctionnel, vendu sous la dénomination CARBOPOL 940 par la Société GOODRICH 0,80 g
- NaOH 0,125 g
- Eau qsp 100,00 g

Cette composition se présente sous forme d'un gel.

On constate que lorsque ces compositions sont appliquées sur une peau présentant les signes cliniques d'un vieillissement, l'aspect de celles-ci prend une couleur plus rosée, les rides s'atténuent, les tâches telles que le lentigo sénile, disparaissent.

Les résultats permettent de conclure à l'effet anti-vieillissement de ces compositions.

Ces compositions ont également permis de constater, par mesure du pli cutané sur des souris mâles OLAC, une nette augmentation de l'épaisseur de ce pli à la suite d'un traitement consistant en l'application de ces compositions à raison de 5 jours par semaine pendant deux mois 1/2.

## Revendications

1. Utilisation d'une composition cosmétique contenant dans un milieu physiologiquement acceptable, au moins un dérivé d'acide salicylique répondant à la formule : 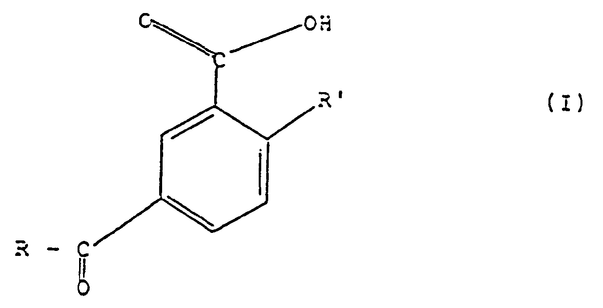 dans laquelle :
R représente une chaîne aliphatique saturée ayant de 3 à 11 atomes de carbone, linéaires, ramifiée ou cyclisée, une chaîne insaturée ayant de 3 à 17 atomes de carbone, portant une ou plusieurs doubles liaisons conjuguées ou non, les chaînes pouvant être substituées par un ou plusieurs atomes d'halogène ainsi que par des groupements trifluorométhyle, par un ou plusieurs groupement hydroxyle sous forme libre ou estérifiée par un acide ayant de 1 à 6 atomes de carbone ou bien par une fonction carboxyle, libre ou estérifiée, par un alcool inférieur ayant de 1 à 6 atomes de carbone, ces différents groupements pouvant être simultanément présents sur lesdits substituants;
R' représente un groupement hydroxyle ou une fonction ester de formule : 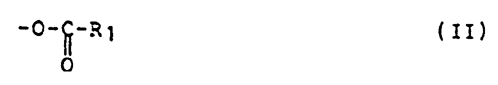 où R₁ est un groupement aliphatique, saturé ou insaturé, ayant de 1 à 18 atomes de carbone, pour atténuer les rides, modifier le teint de la peau, effacer les taches pigmentées en surface, supprimer les squames et/ou conférer à la peau un toucher plus doux.

2. Utilisation selon la revendication 1, caractérisée par le fait que les composés de formule (I) sont choisis parmi les composés dans lesquels R' désigne une fonction hydroxyle et R désigne un groupement alkyle ayant de 3 à 11 atomes de carbone.

3. Utilisation selon la revendication 1, caractérisé par le fait que dans le composé de formule (I), R représente une chaîne dérivée de l'acide linoléique, linolénique ou oléique.

4. Utilisation selon la revendication 1, caractérisée par le fait que dans le composé de formule (I), R désigne une chaîne alkyle ayant de 3 à 11 atomes de carbone et portant un groupement carboxylique libre, estérifié ou salifié, et R' représente une fonction hydroxyle.

5. Utilisation selon la revendication 1, caractérisée par le fait que le composé de formule (I) est choisi parmi l'acide n-octanoyl-5 salicylique, l'acide n-dodécanoyl-5 salicylique, l'acide n-décanoyl-5 salicylique.

6. Utilisation selon l'une quelconque des revendications 1 à 5, caractérisée par le fait que la composition se présente sous forme de gel, de crème, de lait, de stick, de mousse, de savon, d'huile, de composition grasse ou de lotion pouvant être pressurisée dans un dispositif aérosol.

7. Utilisation selon l'une quelconque des revendications 1 à 6, caractérisé par le fait que le composé de formule (I) est présent dans la composition dans des proportions comprises entre 0,1 et 10% en poids par rapport au poids total de la composition.

8. Utilisation selon l'une quelconque des revendications 1 à 7, caractérisé par le fait que la composition contient en plus des épaississants, des adoucissants, des surgraissants, des émollients, des mouillants, des agents de surface, des conservateurs, des agents anti-mousses, des filtres solaires, des huiles, des cires, des colorants et/ou des pigments ayant pour fonction de colorer la composition ou la peau, des anti-oxydants et tout autre ingrédient habituellement utilisé dans des compositions destinées à l'application topique.

9. Utilisation selon l'une quelconque des revendications 1 à 8, caractérisée par le fait que la composition contient en plus des rétinoïdes.

10. Utilisation selon l'une quelconque des revendications 1 à 9, caractérisée par le fait que la composition se présente sous forme d'un gel anhydre, contenant un agent épaississant, un alcool inférieur et un polyalcool.

11. Utilisation selon l'une quelconque des revendications 1 à 9, caractérisée par le fait que la composition se présente sous forme d'une pommade grasse.

12. Utilisation selon l'une quelconque des revendications 1 à 9, caractérisé par le fait que la composition se présente sous forme d'une crème.

13. Procédé de traitement cosmétique du vieillissement de la peau, pour atténuer les rides, modifier le teint de la peau, effacer les taches pigmentées en surface, supprimer les squames et/ou conférer à la peau un toucher plus doux caractérisé par le fait que l'on applique sur la peau au moins une composition cosmétique contenant dans un milieu physiologiquement acceptable, au moins un dérivé d'acide salicylique répondant à la formule : 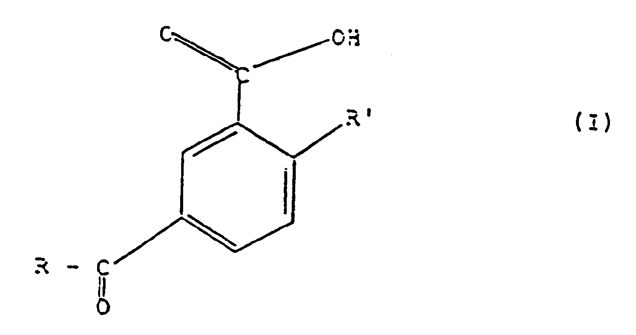 dans laquelle :
R représente une chaîne aliphatique saturée ayant de 3 à 11 atomes de carbone, linéaire, ramifiée ou cyclisée, une chaîne insaturée ayant de 3 à 17 atomes de carbone, portant une ou plusieurs doubles liaisons conjuguées ou non, les chaînes pouvant être substituées par un ou plusieurs atomes d'halogène ainsi que par des groupements trifluorométhyle, par un ou plusieurs groupements hydroxyle sous forme libre ou estérifiée par un acide ayant de 1 à 6 atomes de carbone ou bien par une fonction carboxyle, libre ou estérifiée, par un alcool inférieur ayant de 1 à 6 atomes de carbone, ces différents groupements pouvant être simultanément présents sur lesdits substituants;
R' représente un groupement hydroxyle ou une fonction ester de formule : 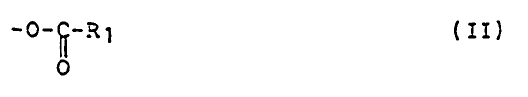 où R₁ est un groupement aliphatique, saturé ou insaturé, ayant de 1 à 18 atomes de carbone

14. Utilisation d'une composition contenant dans un milieu physiologiquement acceptable, au moins un dérivé d'acide salicylique répondant à la formule : 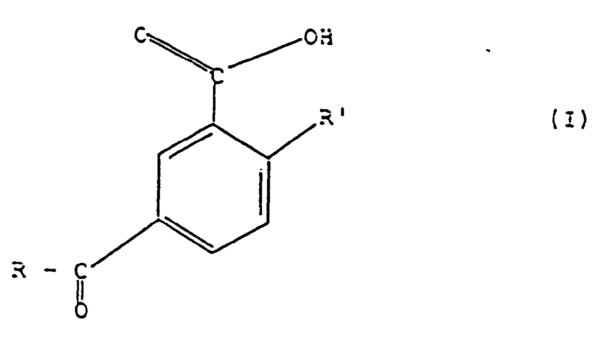 dans laquelle:
R représente une chaîne aliphatique saturée ayant de 3 à 11 atomes de carbone, linéaire, ramifiée ou cyclisée, une chaîne insaturée ayant de 3 à 17 atomes de carbone, portant une ou plusieurs doubles liaisons conjuguées ou non, les chaînes pouvant être substituées par un ou plusieurs atomes d'halogène ainsi que par des groupements trifluorométhyle, par un ou plusieurs groupements hydroxyle sous forme libre ou estérifiée par un acide ayant de 1 à 6 atomes de carbone ou bien par une fonction carboxyle, libre ou estérifiée, par un alcool inférieur ayant de 1 à 6 atomes de carbone, ces différents groupements pouvant être simultanément présents sur lesdits substituants;
R' représente un groupement hydroxyle ou une fonction ester de formule : 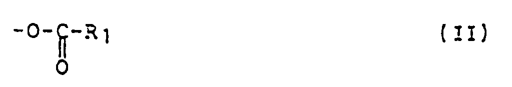 où R₁ est un groupement aliphatique, saturé ou insaturé, ayant de 1 à 18 atomes de carbone,
pour la préparation d'un médicament destiné au traitement thérapeutique du vieillissement de la peau.

## Patentansprüche

1. Verwendung einer kosmetischen Zusammensetzung, die in einem physiologisch annehmbaren Milieu mindestens ein Derivat einer Salicylsäure der Formel: 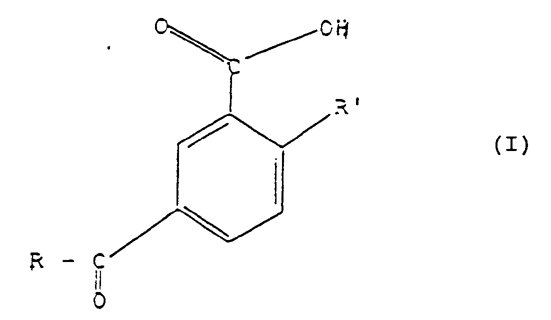 enthält, worin bedeuten:
R₁, eine lineare, verzweigte oder cyclisierte Kohlenstoffkette mit 1 bis 11 Kohlenstoffatomen, eine ungesättigte Kohlenstoffkette mit 3 bis 17 Kohlenstoffatomen, die eine oder mehrere Doppelbindungen, die konjugiert sein können oder nicht, enthalten, und die Ketten durch eine oder mehrere Halogenatome, wie z.B. Trifluormethylgruppen, durch eine oder mehrere Hydroxylgruppen in freier Form oder in veresterter Form mit einer Säure mit 1 bis 6 Kohlenstoffatomen oder durch eine freie oder durch einen niederen Alkohol mit 1 bis 6 Kohlenstoffatomen veresterte Carboxylgruppe substituiert sein können, wobei die verschiedenen Gruppen gleichzeitig die Substituenten tragen können;
R' eine Hydroxylgruppe oder eine Estergruppe der Formel: 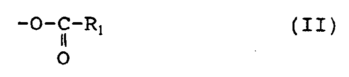 bedeutet, worin R₁ eine aliphatische gesättigte oder ungesättigte Gruppe ist, die 1 bis 18 Kohlenstoffatomen besitzt, um die Falten zu mildern, den Teint der Haut zu modifizieren und die Pigmentflecken auf der Oberfläche zu unterdrucken und der Haut einen weichen Griff zu verleihen.

2. Verwendung nach Anspruch 1, dadurch gekennzeichnet, daß die Verbindungen der Formel (I) ausgewählt sind aus den Verbindungen, in denen R' eine Hydroxylgruppe bedeutet, und R eine Alkylgruppe mit 1 bis 11 Kohlenstoffatomen.

3. Verwendung gemäß Anspruch 1, dadurch gekennzeichnet, daß in der Zusammensetzung der Formel (I) R eine Kette bedeutet, abgeleitet von Linolsäure, Linolensäure oder Ölsäure.

4. Verwendung nach Anspruch 1, dadurch gekennzeichnet, daß in der Zusammensetzung der Formel (I) R eine Alkylkette mit 1 bis 3 Kohlenstoffatomen bedeutet und eine freie Carboxylgruppe trägt, die verestert oder versalzt sein kann, und R' eine Hydroxylgruppe darstellt.

5. Verwendung nach Anspruch 1, dadurch gekennzeichnet, daß die Verbindung der Formel (I) ausgewählt ist aus 5-n-Octanoyl-salicylsäure, 5-n-Dodecanoyl-salicylsäure, 5-n-Decanoyl-salicylsäure.

6. Verwendung gemäß einem der Ansprüche 1 bis 5, dadurch gekennzeichnet, daß die Zusammensetzung in Form eines Gels, einer Creme, einer Milch, eines Stiftes, eines Schaumes, einer Seife, eines Öles, einer fettigen Zusammensetzung oder Lotion vorliegt, die in einem Aerosolbehälter gepreßt sein kann.

7. Verwendung nach einem der Ansprüche 1 bis 6, dadurch gekennzeichnet, daß die Zusammensetzung der Formel (I) in einer Zusammensetzung vorliegt, deren Anteile zwischen 0.1 bis 10 Gew.-%, bezogen auf das Gesamtgewicht der Zusammensetzung vorliegen.

8. Zusammensetzung gemäß einem der Ansprüche 1 bis 7, dadurch gekennzeichnet, daß die Zusammensetzung darüberhinaus enthält Verdickungsmittel, Weichmacher, Seifen, Emollientien, Feuchthaltemittel, oberflächenaktive Mittel, Konservierungsstoffe, Antischaummittel, Sonnenfilter, Öle, Wachse, Farbstoffe und/oder Pigmente zur Färbung der Zusammensetzung oder der Haut, Antioxidantien und andere gebräuchliche Bestandteile, wie sie in Zusammensetzungen für die topische Applikation verwendet werden.

9. Verwendung nach einem der Ansprüche 1 bis 8, dadurch gekennzeichnet, daß die Zusammensetzungen außerdem Retinoide enthalten.

10. Verwendung nach einem der Ansprüche 1 bis 9, dadurch gekennzeichnet, daß die Zusammensetzung in Form eines wasserfreien Gels vorliegt, das ein Verdickungsmittel enthält, einen niederen Alkohol und einen Polyalkohol.

11. Verwendung nach einem der Ansprüche 1 bis 9, dadurch gekennzeichnet, daß die Zusammensetzung in Form einer Fettsalbe vorliegt.

12. Verwendung nach einem der Ansprüche 1 bis 9, dadurch gekennzeichnet, daß die Zusammensetzung in Form einer Creme vorliegt.

13. Verfahren zur kosmetischen Behandlung gegenüber dem Altern der Haut zur Milderung der Falten, zur Modifizierung des Teints der Haut, und zum Auslöschen und zur Unterdrückung der Pickel der Haut und/oder um sie mit einer Weichheit auszustatten, dadurch gekennzeichnet, daß man auf die Haut mindestens eine kosmetische Zusammensetzung appliziert, die in einem physiologisch annehmbaren Milieu mindestens ein Derivat einer Salicylsäure der folgenden Formel 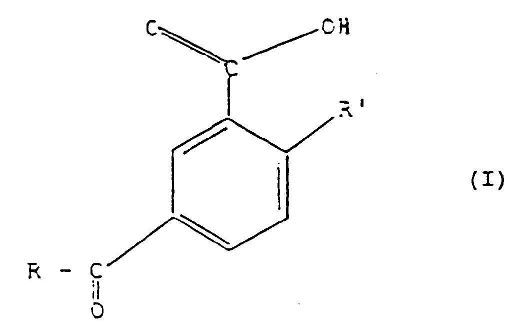 enthält, worin bedeuten:
R eine gesättigte aliphatische lineare, verzweigte oder cyclische Kette mit 3 bis 11 Kohlenstoffatomen, eine ungesättigte Kette mit 3 bis 17 Kohlenstoffatomen, die ein oder mehrere Doppelbindungen tragen kann, die in Konjugation oder nicht in Konjugation zueinander sind, wobei die Ketten durch eine oder mehrere Halogenatome, wie z.B. die Trifluormethylgruppe substituiert sein können, oder durch eine oder mehrere Hydroxylgruppen in freier Form oder mit einer Säure mit 1 bis 6 Kohlenstoffatomen verestert, oder durch eine freie oder durch einen niederen Alkohol mit 1 bis 6 Kohlenstoffatomen veresterte Carboxylgruppe, wobei die verschiedenen Gruppen gleichzeitig als Substituenten vorhanden sein können; R' eine Hydroxylgruppe oder eine Estergruppe der Formel: 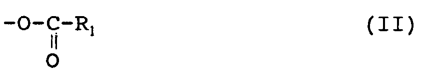 worin R₁ eine aliphatische gesättigte oder ungesättigte Gruppe mit 1 bis 18 Kohlenstoffatomen sein kann.

14. Verwendung einer Zusammensetzung, die in einem physiologisch annehmbaren Milieu mindestens ein Derivat der Salicylsäure der folgenden Formel enthält: 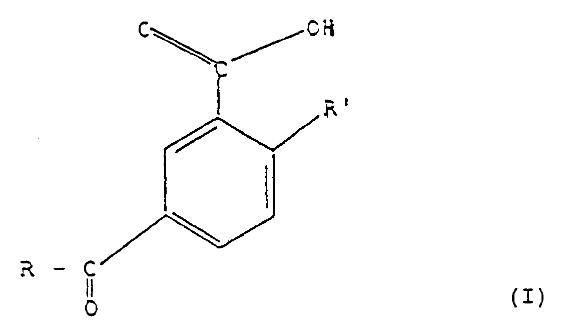 worin bedeuten:
R eine aliphatische gesättigte lineare, verzweigte oder cyclische Kette mit 3 bis 11 Kohlenstoffatomen, eine ungesättigte Kette mit 3 bis 17 Kohlenstoffatomen, die ein oder mehrere Doppelbindungen tragen kann, die konjugiert oder nicht konjugiert sind, wobei die Ketten durch eine oder mehrere Halogenatome substituiert sein können, wie z.B. durch die Trifluormethylgruppe, oder durch eine oder mehrere Hydroxylgruppen in freier oder in mit einer Säure mit 1 bis 6 Kohlenstoffatomen esterifizierten Form, oder durch eine freie oder mit einem niederen Alkohol mit 1 bis 6 Kohlenstoffatomen esterifizierten Carboxylgruppe, wobei die verschiedenen Gruppen gleichzeitig als Substituenten vorhanden sein können;
R' eine Hydroxylgruppe oder eine Estergruppe der Formel: 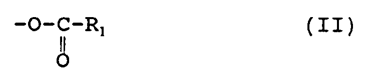 worin R₁ eine aliphatische gesättigte oder ungesättigte Gruppe mit 1 bis 18 Kohlenstoffatomen bedeutet, zur Herstellung eines Medikamentes für die therapeutische Behandlung der Alterung der Haut.

## Claims

1. Use of a cosmetic composition containing, in a physiologically acceptable medium, at least one salicylic acid derivative of the formula: 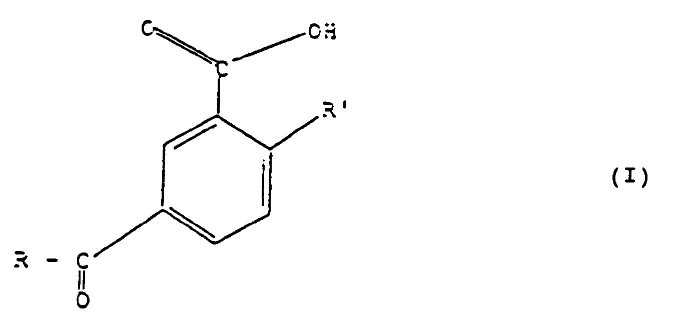 in which:
R represents a linear, branched or cyclised saturated aliphatic chain having 3 to 11 carbon atoms, an unsaturated chain having 3 to 17 carbon atoms having one or more conjugated or unconjugated double bonds, it being possible for the chains to be substituted by one or more halogen atoms as well as by trifluoromethyl groups, by one or more hydroxyl groups in a free form or esterified by an acid having 1 to 6 carbon atoms or alternatively by a carboxyl functional group which is free or esterified by a lower alcohol having 1 to 6 carbon atoms, it being possible for these various groups to be simultaneously present on the said substituents;
R' represents a hydroxyl group or an ester functional group of formula: 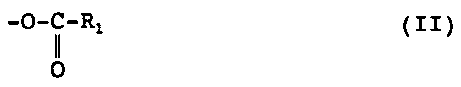 where R₁ is a saturated or unsaturated aliphatic group having 1 to 18 carbon atoms, for attenuating wrinkles, modifying skin complexion, removing superficially pigmented spots, suppressing scales and/or conferring a softer feel on the skin.

2. Use according to Claim 1, characterised in that the compounds of formula (I) are chosen from compounds in which R' denotes a hydroxyl functional group and R denotes an alkyl group having 3 to 11 carbon atoms.

3. Use according to Claim 1, characterised in that in the compound of formula (I), R represents a chain derived from linoleic acid, linolenic or oleic acid.

4. Use according to Claim 1, characterised in that in the compound of formula (I), R denotes an alkyl chain having 3 to 11 carbon atoms and having a free, esterified or salified carboxylic group, and R' represents a hydroxyl functional group.

5. Use according to Claim 1, characterised in that the compound of formula (I) is chosen from 5-(n-octanoyl)salicylic acid, 5-(n-dodecanoyl)salicylic acid, 5-(n-decanoyl)salicylic acid.

6. Use according to any one of Claims 1 to 5, characterised in that the composition is provided in the form of a gel, cream, milk, stick, foam, soap, oil, fatty composition or lotion which can be packaged under pressure in an aerosol device.

7. Use according to any one of Claims 1 to 6, characterised in that the compound of formula (I) is present in the composition in proportions of between 0.1 and 10 % by weight relative to the total weight of the composition.

8. Use according to any one of Claims 1 to 7, characterised in that the composition additionally contains thickeners, demulcents, superfatting agents, emollients, wetting agents, surface-active agents, preservatives, antifoaming agents, sunscreen agents, oils, waxes, colorants and/or pigments designed for colouring the composition or the skin, antioxidants and any other ingredients normally used in compositions intended for topical application.

9. Use according to any one of Claims 1 to 8, characterised in that the composition additionally contains retinoids.

10. Use according to any one of Claims 1 to 9, characterised in that the composition is provided in the form of an anhydrous gel containing a thickening agent, a lower alcohol and a polyalcohol.

11. Use according to any one of Claims 1 to 9, characterised in that the composition is provided in the form of a fatty ointment.

12. Use according to any one of Claims 1 to 9, characterised in that the composition is provided in the form of a cream.

13. Process for the cosmetic treatment of skin ageing, for attenuating wrinkles, modifying skin complexion, removing superficially pigmented spots, suppressing scales and/or conferring a softer feel on the skin, characterised in that there is applied to the skin at least one cosmetic composition containing, in a physiologically acceptable medium, at least one salicylic acid derivative of the formula: 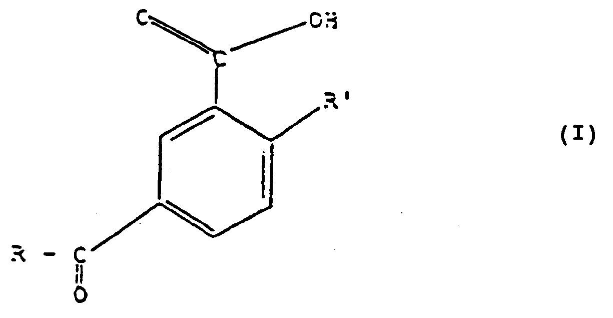 in which:
R represents a linear, branched or cyclised saturated aliphatic chain having 3 to 11 carbon atoms, an unsaturated chain having 3 to 17 carbon atoms having one or more conjugated or unconjugated double bonds, it being possible for the chains to be substituted by one or more halogen atoms as well as by trifluoromethyl groups, by one or more hydroxyl groups in a free form or esterified by an acid having 1 to 6 carbon atoms or alternatively by a carboxyl functional group which is free or esterified by a lower alcohol having 1 to 6 carbon atoms, it being possible for these various groups to be simultaneously present on the said substituents;
R' represents a hydroxyl group or an ester functional group of formula: 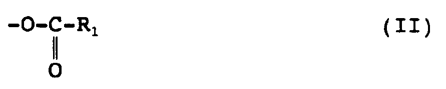 where R₁ is a saturated or unsaturated aliphatic group having 1 to 18 carbon atoms.

14. Use of a composition containing, in a physiologically acceptable medium, at least one salicylic acid derivative of the formula: 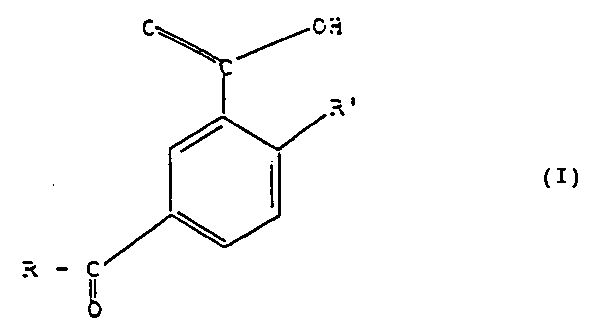 in which:
R represents a linear, branched or cyclised saturated aliphatic chain having 3 to 11 carbon atoms, an unsaturated chain having 3 to 17 carbon atoms having one or more conjugated or unconjugated double bonds, it being possible for the chains to be substituted by one or more halogen atoms as well as by trifluoromethyl groups, by one or more hydroxyl groups in a free form or esterified by an acid having 1 to 6 carbon atoms or alternatively by a carboxyl functional group which is free or esterified by a lower alcohol having 1 to 6 carbon atoms, it being possible for these various groups to be simultaneously present on the said substituents;
R' represents a hydroxyl group or an ester functional group of formula: 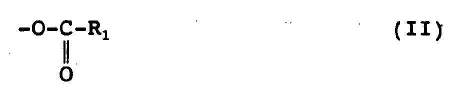 where R₁ is a saturated or unsaturated aliphatic group having 1 to 18 carbon atoms, for preparing a medicinal product intended for the therapeutic treatment of skin ageing.
